(19) Europäisches Patentamt
European Patent Office
Office européen des brevets

(11) EP 4 389 735 A1

(12) EUROPEAN PATENT APPLICATION

(43) Date of publication:
**26.06.2024 Bulletin 2024/26**

(21) Application number: **23220171.5**

(22) Date of filing: **22.12.2023**

(51) International Patent Classification (IPC):
***C07C 69/00*** *(2006.01)* ***C07C 211/01*** *(2006.01)*
***C08K 5/00*** *(2006.01)* ***C08K 5/17*** *(2006.01)*

(52) Cooperative Patent Classification (CPC):
(C-Sets available)
**C08K 5/0016; C07C 229/24; C08K 5/175;**
C08L 2201/06 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.12.2022 TW 111149575**

(71) Applicant: **Largan Medical Co., Ltd.**
**Taichung City 408 (TW)**

(72) Inventors:
- **CHEN, Po-Tsun**
**408 Taichung City (TW)**
- **LU, Tzu-Rong**
**408 Taichung City (TW)**
- **CHEN, Rih-Sian**
**408 Taichung City (TW)**
- **HONG, Yu Jie**
**408 Taichung City (TW)**
- **TENG, Chun-Hung**
**408 Taichung City (TW)**

(74) Representative: **Lang, Christian**
**LangPatent Anwaltskanzlei IP Law Firm**
**Ingolstädter Straße 5**
**80807 München (DE)**

(54) **PLASTICIZER, PLASTIC COMPOSITION, AND PLASTIC PRODUCT**

(57) A plasticizer, a plastic composition and a plastic product are provided. The plasticizer is an amino acid dérivative and has a biodegradability, wherein the plasticizer includes a central structure and at least one side chain structure. The central structure is an aspartic acid, the at least one side chain structure includes a first side chain structure and a second side chain structure. The plasticizer has a structure represented by Formula (I), in which each of the symbols thereof is as defined in the spécification.

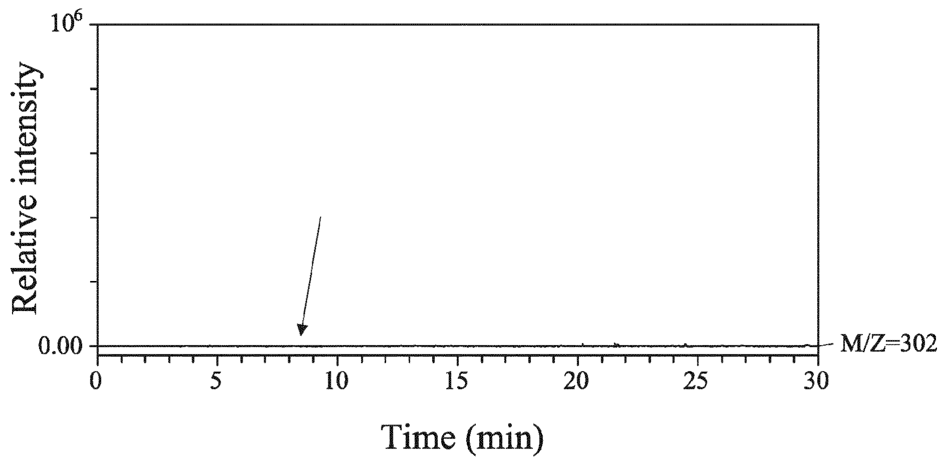


Fig. 2

(52) Cooperative Patent Classification (CPC): (Cont.)

C-Sets
**C08K 5/175, C08L 67/04;**
**C08L 67/04, C08L 67/02, C08K 5/175**

## Description

### BACKGROUND

Technical Field

The present disclosure relates to a plasticizer, a plastic composition and a plastic product. More particularly, the present disclosure relates to a plasticizer, a plastic composition and a plastic product that have a biodegradability.

Description of Related Art

The plasticizer is a material used to increase the plasticity of the product, and it is often used in the plasticization of the plastic product. The commonly used traditional plasticizers are mostly phthalate compounds with structures similar to estrogen, such as DEHP, DBP, DINP, BBP, DIDP, etc. The aforementioned plasticizers are non-biodegradable and can cause the abnormal development of adolescent children easily, and even increase the incidence of the breast cancer and the ovarian cancer. Since the plasticizers are combined with other substances by the non-chemical bonding method in the plastic products, so that the plasticizers are easily released from the plastic products. Therefore, the plasticizers will be absorbed by the human body when the plastic products contact with food or skin.

In the recent years, the environmental protection awareness has risen, and if the traditional plastic products are not properly treated, they will damage the environment and the ecology. Thus, more and more biodegradable products are widely promoted, but the biodegradation rate thereof is often not high. Most plasticizers and plastics still cannot be decomposed, and the users may misunderstand that these products will not affect the environment and the ecology by any disposal due to these products are biodegradable, resulting in these products not being properly treated and further damaging the environment and the ecology. Nowadays, the plastic products have become an indispensable part of human life, so that it is very important to find the plasticizers that are not easily released from the plastic products or absorbed by the human body, and to develop the biodegradable plastic products.

### SUMMARY

According to one aspect of the present disclosure, a plasticizer is an amino acid derivative and has a biodegradability. The plasticizer includes a central structure and at least one side chain structure, wherein the central structure is an aspartic acid, and the at least one side chain structure includes a first side chain structure and a second side chain structure. The plasticizer has a structure represented by Formula (I):

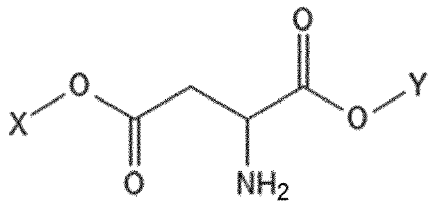

Formula (I),

wherein X is the first side chain structure, Y is the second side chain structure, a number of carbon atoms of the first side chain structure is XC, a number of carbon atoms of the second side chain structure is YC, and the following conditions are satisfied: $1 \leq XC \leq 7$; and $1 \leq YC \leq 7$.

According to the plasticizer of the foregoing aspect, at least one of X and Y can be a saturated hydrocarbon chain.

According to the plasticizer of the foregoing aspect, at least one of X and Y can be a branched chain structure.

According to the plasticizer of the foregoing aspect, a number of carbon atoms of X can be different from a number of carbon atoms of Y.

According to the plasticizer of the foregoing aspect, at least one of X and Y can have a hydroxyl group.

According to the plasticizer of the foregoing aspect, when a molecular weight of the plasticizer is Mw, the following condition can be satisfied: 300 g/mole $\leq$ Mw.

According to the plasticizer of the foregoing aspect, when a pyrolysis temperature of the plasticizer is Pt, the following condition can be satisfied: 100°C $\leq$ Pt.

According to the plasticizer of the foregoing aspect, when a biodegradation rate on a 14th day is Dr14, the

following condition of the biodegradation rate on the 14th day of the plasticizer can be satisfied: Dr14 ≤ 15%.

According to the plasticizer of the foregoing aspect, when a biodegradation rate on a 35th day is Dr35, the following condition of the biodegradation rate on the 35th day of the plasticizer can be satisfied: Dr35 ≤ 45%.

According to the plasticizer of the foregoing aspect, when a biodegradation rate on a 180th day is Dr180, the following condition of the biodegradation rate on the 180th day of the plasticizer can be satisfied: 85% ≤ Dr180.

According to the plasticizer of the foregoing aspect, a cell viability can be greater than or equal to 70% when a concentration of the plasticizer is 0.3 mM.

According to another aspect of the present disclosure, a plastic composition includes at least one plastic and the plasticizer according to the foregoing aspect.

According to the plastic composition of the foregoing aspect, a weight percentage of the at least one plastic in the plastic composition can be 85.00% to 99.99%, and a weight percentage of the plasticizer in the plastic composition can be 0.01% to 15.00%.

According to the plastic composition of the foregoing aspect, the at least one plastic can include a biodegradable plastic.

According to the plastic composition of the foregoing aspect, the at least one plastic can further include at least one non-biodegradable plastic, wherein a weight percentage of the at least one non-biodegradable plastic in the plastic composition can be less than a weight percentage of the biodegradable plastic in the plastic composition.

According to further another aspect of the present disclosure, a plastic product includes the plastic composition according to the foregoing aspect.

According to still another aspect of the present disclosure, a plasticizer is an amino acid derivative and has a biodegradability. The plasticizer includes a central structure and at least one side chain structure, wherein the central structure is an aspartic acid, and the at least one side chain structure includes a first side chain structure and a second side chain structure. The plasticizer has a structure represented by Formula (I):

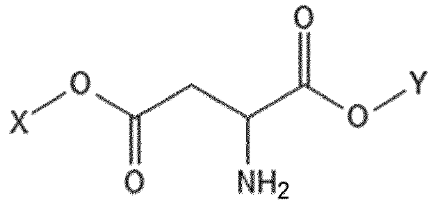

Formula (I),

wherein X is the first side chain structure, Y is the second side chain structure, and at least one of X and Y is a branched chain structure.

According to the plasticizer of the foregoing aspect, when a number of carbon atoms of the first side chain structure is XC, a number of carbon atoms of the second side chain structure is YC, the following conditions can be satisfied: 1 ≤ XC ≤ 10; and 1 ≤ YC ≤ 10.

According to the plasticizer of the foregoing aspect, at least one of X and Y can be a saturated hydrocarbon chain.

According to the plasticizer of the foregoing aspect, when a biodegradation rate on a 14th day is Dr14, the following condition of the biodegradation rate on the 14th day of the plasticizer can be satisfied: Dr14 ≤ 15%.

According to the plasticizer of the foregoing aspect, when a biodegradation rate on a 35th day is Dr35, the following condition of the biodegradation rate on the 35th day of the plasticizer can be satisfied: Dr35 ≤ 45%.

According to the plasticizer of the foregoing aspect, when a biodegradation rate on a 180th day is Dr180, the following condition of the biodegradation rate on the 180th day of the plasticizer can be satisfied: 85% ≤ Dr180.

According to yet another aspect of the present disclosure, a plastic composition includes at least one plastic and at least one plasticizer. The plasticizer is an amino acid derivative and has a biodegradability, and the plasticizer includes a central structure and at least one side chain structure. A weight percentage of the at least one plastic in the plastic composition is 85.00% to 99.99%, and a weight percentage of the plasticizer in the plastic composition is 0.01% to 15.00%.

According to the plastic composition of the foregoing aspect, at least one of the at least one side chain structure can be a long chain structure with a number of carbon atoms ranging from 1 to 10.

According to the plastic composition of the foregoing aspect, at least one of the at least one side chain structure can be a saturated hydrocarbon chain.

According to the plastic composition of the foregoing aspect, at least one of the at least one side chain structure can be a branched chain structure.

According to the plastic composition of the foregoing aspect, when a biodegradation rate on a 14th day is Dr14, the following condition of the biodegradation rate on the 14th day of the plasticizer can be satisfied: Dr14 ≤ 15%.
According to the plastic composition of the foregoing aspect, when a biodegradation rate on a 35th day is Dr35, the following condition of the biodegradation rate on the 35th day of the plasticizer can be satisfied: Dr35 ≤ 45%.
According to the plastic composition of the foregoing aspect, when a biodegradation rate on a 180th day is Dr180, the following condition of the biodegradation rate on the 180th day of the plasticizer can be satisfied: 85% ≤ Dr180.

**BRIEF DESCRIPTION OF THE DRAWINGS**

The present disclosure can be more fully understood by reading the following detailed description of the embodiment, with reference made to the accompanying drawings as follows:

Fig. 1 shows the results of LS-MS of the control group A.

Fig. 2 shows the results of LS-MS of the experiment group E.

**DETAILED DESCRIPTION**

The present disclosure provides a plasticizer, which is an amino acid derivative and has a biodegradability. The plasticizer includes a central structure and at least one side chain structure. The central structure is an aspartic acid, and the at least one side chain structure includes a first side chain structure and a second side chain structure. The plasticizer has a structure represented by Formula (I):

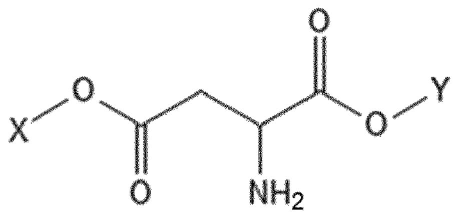

Formula (I),

wherein X is the first side chain structure, and Y is the second side chain structure. Therefore, the damage to the environment and the ecology can be reduced by using the biodegradable plasticizer. Further, the plasticizer can have the excellent safety to prevent it from affecting the normal physiological functions of the human body by being designed without a phthalate structure, which is different from the traditional non-biodegradable plasticizer. Furthermore, the aspartic acid is a kind of the amino acid, which has the characteristics of biodegradable and biometabolizable, so that the plasticizer can have the better safety by designing the chemical structure based on the aspartic acid.
According to the plasticizer of the present disclosure, at least one of the at least one side chain structure is a long chain structure with a number of carbon atoms ranging from 1 to 10, and thus in the Formula (I), a number of carbon atoms of the first side chain structure is XC, a number of carbon atoms of the second side chain structure is YC, and the following conditions are satisfied: 1 ≤ XC ≤ 7; and 1 ≤ YC ≤ 7. Therefore, it is favorable for the plasticizer to have an excellent biodegradation rate by satisfying the number of carbon atoms of the first side chain structure and the second side chain structure in a proper range. Furthermore, the following conditions can be satisfied: 1 ≤ XC ≤ 10; and 1 ≤ YC ≤ 10. Furthermore, the following conditions can be satisfied: 2 ≤ XC ≤ 9; and 2 ≤ YC ≤ 9. Furthermore, the following conditions can be satisfied: 3 ≤ XC ≤ 8; and 3 ≤ YC ≤ 8. Furthermore, the following conditions can be satisfied: 4 ≤ XC ≤ 7; and 4 ≤ YC ≤ 7. Furthermore, the following conditions can be satisfied: 5 ≤ XC ≤ 6; and 5 ≤ YC ≤ 6.
According to the plasticizer of the present disclosure, at least one of the at least one side chain structure is a saturated hydrocarbon chain, and thus in the Formula (I), at least one of X and Y is the saturated hydrocarbon chain. Therefore, it is favorable for increasing the stability of the plasticizer structure by designing the side chain structure with the saturated hydrocarbon chain.
According to the plasticizer of the present disclosure, at least one of the at least one side chain structure is a branched chain structure, and thus in the Formula (I), at least one of X and Y is the branched chain structure. Therefore, it is favorable for increasing the strength of the plasticizer after mixing with other compounds by designing the side chain structure with the branched chain structure.
According to the plasticizer of the present disclosure, in the Formula (I), a number of carbon atoms of X is

different from a number of carbon atoms of Y. Therefore, it is favorable for reducing the difficulty of the plasticizer mixing with other compounds by designing the side chain structures with different carbon numbers.

According to the plasticizer of the present disclosure, in the Formula (I), at least one of X and Y has a hydroxyl group. Therefore, it is favorable for improving the biodegradation rate of the plasticizer by designing the structure with the hydroxyl group.

According to the plasticizer of the present disclosure, when a molecular weight of the plasticizer is Mw, the following condition can be satisfied: 300 g/mole ≤ Mw. Therefore, the plasticizer is not easily absorbed by the human body and can have the excellent safety by designing the plasticizer with the specific molecular weight. Furthermore, the following condition can be satisfied: 100 g/mole ≤ Mw. Furthermore, the following condition can be satisfied: 150 g/mole ≤ Mw. Furthermore, the following condition can be satisfied: 200 g/mole ≤ Mw ≤ 1000 g/mole. Furthermore, the following condition can be satisfied: 250 g/mole ≤ Mw ≤ 800 g/mole. Furthermore, the following condition can be satisfied: 300 g/mole ≤ Mw ≤ 500 g/mole.

According to the plasticizer of the present disclosure, when a pyrolysis temperature of the plasticizer is Pt, the following condition can be satisfied: 100°C ≤ Pt. Therefore, it is favorable for stabilizing the structure of the plasticizer at the high temperature by the high pyrolysis temperature of the plasticizer. Furthermore, the following condition can be satisfied: 120°C ≤ Pt. Furthermore, the following condition can be satisfied: 150°C ≤ Pt. Furthermore, the following condition can be satisfied: 170°C ≤ Pt. Furthermore, the following condition can be satisfied: 180°C ≤ Pt.

According to the plasticizer of the present disclosure, when a biodegradation rate on a 14th day is Dr14, the following condition of the biodegradation rate on the 14th day of the plasticizer can be satisfied: Dr14 ≤ 15%. Therefore, it is favorable for improving the service life of the plasticizer by limiting the biodegradation rate on the 14th day of the plasticizer. Furthermore, the following condition can be satisfied: Dr14 ≤ 20%. Furthermore, the following condition can be satisfied: Dr14 ≤ 25%. Furthermore, the following condition can be satisfied: Dr14 ≤ 30%. Furthermore, the following condition can be satisfied: Dr14 ≤ 35%. Furthermore, the following condition can be satisfied: 0% ≤ Dr14 ≤ 40%.

According to the plasticizer of the present disclosure, when a biodegradation rate on a 35th day is Dr35, the following condition of the biodegradation rate on the 35th day of the plasticizer can be satisfied: Dr35 ≤ 45%. Therefore, the service life of the plasticizer can be further improved by limiting the biodegradation rate on the 35th day of the plasticizer. Furthermore, the following condition can be satisfied: Dr35 ≤ 50%. Furthermore, the following condition can be satisfied: 0% ≤ Dr35 ≤ 55%.

According to the plasticizer of the present disclosure, when a biodegradation rate on a 180th day is Dr180, the following condition of the biodegradation rate on the 180th day of the plasticizer can be satisfied: 85% ≤ Dr180. Therefore, it is favorable for improving the final biodegradation rate of the plasticizer by satisfying the biodegradation rate on the 180th day of the plasticizer. Furthermore, the following condition can be satisfied: 87.5% ≤ Dr180. Furthermore, the following condition can be satisfied: 90% ≤ Dr180. Furthermore, the following condition can be satisfied: 92.5% ≤ Dr180 ≤ 100%.

According to the plasticizer of the present disclosure, when a concentration of the plasticizer is 0.1 mM, a cell viability is greater than or equal to 70%. Therefore, it is favorable for increasing the safety of the plasticizer by limiting the cell viability to ensure the plasticizer is non-cytotoxic. Furthermore, when the concentration of the plasticizer is 0.1 mM, the cell viability can be greater than or equal to 75%. Furthermore, when the concentration of the plasticizer is 0.1 mM, the cell viability can be greater than or equal to 80%. Furthermore, when the concentration of the plasticizer is 0.1 mM, the cell viability can be greater than or equal to 85%. Furthermore, when the concentration of the plasticizer is 0.1 mM, the cell viability can be greater than or equal to 90%.

According to the plasticizer of the present disclosure, when a concentration of the plasticizer is 0.3 mM, a cell viability is greater than or equal to 70%. Therefore, it is favorable for increasing the safety of the plasticizer by limiting the cell viability to ensure the plasticizer is non-cytotoxic. Furthermore, when the concentration of the plasticizer is 0.3 mM, the cell viability can be greater than or equal to 75%. Furthermore, when the concentration of the plasticizer is 0.3 mM, the cell viability can be greater than or equal to 80%.

The present disclosure provides a plastic composition, which includes at least one plastic and the abovementioned plasticizer. Therefore, the damage to the environment and the ecology can be reduced by designing the plastic composition containing the biodegradable plasticizer.

The present disclosure provides a plastic composition, which includes at least one plastic and at least one plasticizer. The plasticizer is an amino acid derivative and has a biodegradability. The plasticizer includes a central structure and at least one side chain structure. Therefore, the better safety can be achieved and the damage to the environment and the ecology can be reduced by designing the biodegradable plasticizer with the amino acid structure.

According to the plastic composition of the present disclosure, a weight percentage of the plastic in the plastic composition is 85.00% to 99.99%, and a weight percentage of the plasticizer in the plastic composition is 0.01 % to 15.00%. Therefore, the plastic composition has the good plasticizing effect by designing a specific mixing ratio. Furthermore, the weight percentage of the plastic in the plastic composition can be 90.00% to 99.99%, and the weight percentage of the plasticizer in the plastic composition can be 0.01% to 10.00%. Furthermore, the weight percentage of the plastic

in the plastic composition can be 92.50% to 99.99%, and the weight percentage of the plasticizer in the plastic composition can be 0.01% to 7.50%. Furthermore, the weight percentage of the plastic in the plastic composition can be 95.00% to 99.99%, and the weight percentage of the plasticizer in the plastic composition can be 0.01% to 5.00%. Furthermore, the weight percentage of the plastic in the plastic composition can be 97.50% to 99.99%, and the weight percentage of the plasticizer in the plastic composition can be 0.01% to 2.50%.

According to the plastic composition of the present disclosure, the plastic can include a biodegradable plastic. Therefore, the damage to the environment and the ecology can be further reduced by the characteristics of biodegradable plastic and plasticizer.

According to the plastic composition of the present disclosure, the plastic can further include at least one non-biodegradable plastic. A weight percentage of the non-biodegradable plastic in the plastic composition is less than a weight percentage of the biodegradable plastic in the plastic composition. Therefore, it is favorable for shaping the plastic composition and improving the stability of the structure by the plastic composition including the non-biodegradable plastic. Further, due to the weight percentage of the non-biodegradable plastic in the plastic composition is less than the weight percentage of the biodegradable plastic in the plastic composition, the plastic composition can be ensured to have a good biodegradation rate.

The plasticizer of the present disclosure can be the amino acid derivative, and the amino acid derivative refers to the compound that uses at least one of an amino group ($-NH_2$ group) and a carboxylic acid group ($-COOH$ group) as a reactive functional group to form a bond when the amino acid performs the chemical reaction. The amino acid refers to 20 kinds of amino acids for the biological cells to synthesize the proteins. The amino acid can be alanine, cysteine, aspartate, glutamate, phenylalanine, glycine, histidine, isoleucine, lysine, leucine, methionine, asparagine, proline, glutamine, arginine, serine, threonine, valine, tryptophan or tyrosine.

According to the plasticizer of the present disclosure, the side chain structure means that the central structure is the amino acid, and the amino acid is a methyl group or a long chain structure that uses the amino group or the carboxylic acid group as the reactive functional group to form the bond. The atoms in the side chain structure can include at least one of the carbon atoms and the oxygen atoms. The side chain structure can be the long chain structure including 1 to 10 carbon atoms, 2 to 9 carbon atoms, 3 to 8 carbon atoms, 4 to 7 carbon atoms or 5 to 6 carbon atoms. The long chain structure can be a saturated hydrocarbon chain or an unsaturated hydrocarbon chain, and the long chain structure can be ethyl group, n-propyl group, isopropyl group, n-butyl group, isobutyl group, n-pentyl group, isopentyl group, n-hexyl group, isohexyl group, n-heptyl group, isoheptyl group, n-octyl group, isooctyl group, n-nonyl group, isononyl group, n-decyl group, isodecyl group and the unsaturated state of the aforementioned hydrocarbon chains, or the carbon-containing long chain including the carbon numbers greater than 10, but the present disclosure is not limited thereto. The plasticizer has at least one side chain structure, also can have at least two side chain structures or at least three side chain structures. The side chain structure can be a straight chain structure or a branched chain structure, and the classification of the straight chain structure and the branched chain structure is based on the bonding method of the carbon atoms as the standard. Counting from the bond connection with the central structure and the side chain structure, if the overall bonding of the carbon atoms on the side chain structure has no branch, then it is the straight chain structure. Counting from the bond connection with the central structure and the side chain structure, if the overall bonding of the carbon atoms on the side chain structure has the branch, then it is the branched chain structure. For example, the side chain structure in which the carboxylic acid group bonded to the no. 2 carbon of the isobutyl group is the branched chain structure. Furthermore, the plasticizer can has the multiple side chain structures, and the number of carbon atoms in the multiple side chain structures can be the same or different. The bonding of the side chain structure can include at least one of the carbon-carbon single bond, the carbon-oxygen single bond and the hydrogen-oxygen bond. The hydrogen-oxygen bond can be the boning of the hydroxyl group, and the hydroxyl group can be located in the middle or at the end of the side chain structure. When the central structure of the plasticizer is the aspartic acid, and two carboxylic acid groups are used as the reactive functional groups to generate two side chain structures, both of the two side chain structures are the branched chain structures, wherein the numbers of carbon atoms in both of the two side chain structures are 6, and the plasticizer can be 1,4-bis(2-ethylbutyl) 2-aminobutanedioate. When the central structure of the plasticizer is the aspartic acid, and two carboxylic acid groups are used as the reactive functional groups to generate two side chain structures, both of the two side chain structures are the straight chain structures, wherein the numbers of carbon atoms in both of the two side chain structures are 1, and the plasticizer can be 1,4-dimethyl 2-aminobutanedioate. When the central structure of the plasticizer is the aspartic acid, and two carboxylic acid groups are used as the reactive functional groups to generate two side chain structures, both of the two side chain structures are the straight chain structures, wherein the numbers of carbon atoms in two side chain structures are 8 and 1, respectively, and the plasticizer can be 1-methyl-4-octyl 2-aminobutanedioate. When the central structure of the plasticizer is the aspartic acid, and two carboxylic acid groups are used as the reactive functional groups to generate two side chain structures, both of the two side chain structures are the branched chain structures, wherein the numbers of carbon atoms in both of the two side chain structures are 6, and the plasticizer can be 1,4-bis(3-methylpentan-3-yl) 2-aminobutanedioate. When the central structure of the plasticizer is the aspartic acid, and two carboxylic acid groups are used as the reactive functional groups to generate

two side chain structures, both of the two side chain structures are the branched chain structures, wherein the numbers of carbon atoms in both of the two side chain structures are 4 and each of the ends of the two side chain structures has a hydroxyl group, and the plasticizer can be 1,4-bis(4-hydroxybutyl) 2-aminobutanedioate, but not limited thereto.

According to the plasticizer of the present disclosure, the bonding formed by the connection between the central structure and the side chain structure does not belong to the central structure and the side chain structure.

According to the plasticizer of the present disclosure, the larger molecular weight means that the molecular weight of the plasticizer is greater than 300 g/mole, which is not limited by the plastic materials, and can plasticize different plastics, so that has more wide applicability and convenience.

According to the plasticizer of the present disclosure, the biodegradation rate thereof is the result of the biodegradation rate test, wherein the condition of the biodegradation rate uses ASTM-D5338 as a standard method. The percentage of biodegradation rate is calculated by a unit of weight. The degradation product and the activated sludge (fertilizer) are mixed with each other in a weight ratio of 1:6, and the water content in the degradation tank is maintained at about 50% by perlite. The degradation tank is placed at 58 ± 2°C to observe for 180 days. The main components of the compost include livestock manure, soybean flour, bagasse, wood meal, wheat bran and mushroom-raising waste bag, and the components of the perlite are shown in Table 1.

Table 1

| Substance | $SiO_2$ | $Al_2O_3$ | $Fe_2O_3$ | CaO | $K_2O$ | $Na_2O$ | MgO | $H_2O$ |
|---|---|---|---|---|---|---|---|---|
| Content (%) | 68~74 | ~12 | 0.5~3.6 | 0.7~1.0 | 2~3 | 4~5 | 0.3 | 2.3~6.4 |

The fresh 0.1 M KOH aqueous solution is prepared during the testing and monitoring process, and dispensed into a 50 mL of PP tank. Then, the PP tank is placed in the degradation tank, and incubated in a temperate box (58 ± 2°C). The released $CO_2$ during the decomposition of the degradation product is absorbed by the KOH aqueous solution. The replacement frequency of KOH is 6 times/week at the initial stage of degradation (1-30 days), and changed to 2 times/week after one month until satisfied with 180 days. The total test time can also be greater than 180 days. The value of the collected KOH is measured with a pH meter and compared with the control group (0.1 M KOH aqueous solution is placed in the temperate box without degradation reaction), and then convert the hydrogen ion concentration. Then, the weight of $CO_2$ is converted by the hydrogen ion concentration and estimated the biodegradation rate. The conversion formula mentioned above is as follows: hydroxyl concentration change = hydroxyl concentration in the control group - hydroxyl concentration in the experimental group = hydrogen ion concentration. Carbon dioxide weight (unit: mg) = hydrogen ion concentration × 44/2 × 50 mL (44 is the molecular weight of carbon dioxide, 2 is the carbon dioxide reaction equilibrium coefficient, and 50 mL is the volume of aqueous solution). Inorganization (decomposition) percentage = carbon dioxide weight change / theoretical carbon dioxide content for complete decomposition of the composition × 100%.

The biodegradation rate for the nth day identified in the present disclosure refers to the number of days that the measured object is placed in the degradation tank with ASTM-D5338 as the standard method, wherein the nth day can be the 7th day, the 8th day, the 9th day, the 10th day, the 14th day, the 21st day, the 24th day, the 25th day, the 26th day, the 28th day, the 29th day, the 30th day, the 31st day, the 35th day, the 42nd day, the 52nd day, the 56th day, the 59th day, the 63rd day, the 66th day, the 70th day, the 73rd day, the 77th day, the 80th day, the 84th day, the 87th day, the 91st day, the 94th day, the 98th day, the 101st day, the 150th day, the 154th day, the 157th day, the 175th day, the 178th day or the 180th day, or can be the biodegradation rate for any day from the 1st day to the 180th day.

The plastic composition of the present disclosure can include the biodegradable plasticizer and the biodegradable plastic, and the non-biodegradable plasticizer and the non-biodegradable plastic are added or replaced as required to achieve the balance of function and biodegradable effect. For example, the plastic composition is composed of the biodegradable plasticizer and the biodegradable plastic, and the non-biodegradable plastic is added, wherein the weight percentage of the non-biodegradable plastic in the plastic composition is less than the weight percentage of the biodegradable plastic in the plastic composition.

The biodegradable plastic in the plastic composition of the present disclosure can be polylactic acid (PLA), and also can be starch, poly(butyleneadipate-co-terephthalate) (PBAT), polybutylene succinate (PBS), poly propylene carbonate (PPC), polyhydroxyalkanoates (PHA), polycaprolactone (PCL), polyvinyl alcohol (PVA), polyglycolide (PGA), polyglycerol sebacate (PGS), polyhydroxybutyrate (PHB) or copolymers or mixtures formed from the above polymer monomers, which is not limited thereto.

The non-biodegradable plastic in the plastic composition of the present disclosure can be poly(ethylene terephthalateco-1,4-cylclohexylenedimethylene terephthalate) (PETG), polyethylene terephthalate (PET), high-density polyethylene (HOPE), polyvinyl chloride (PVC), low-density polyethylene (LDPE), polypropene (PP), polystyrene (PS), polycarbonate (PC), polymethylmethacrylate (PMMA), acrylonitrile butadiene styrene (ABS), cyclic olefin copolymer (COC), melamine resin or copolymers or mixtures formed from the above polymer monomers, which is not limited thereto.

The plastic composition of the present disclosure can further include at least one additive, such as a stabilizer, a lubricant, a static coagulant, a flame retardant, a colorant, a filler, an antistatic agent, a light stabilizer, a foaming agent, a coupling agent, and a flexibilizer. The stabilizer can be calcium stearate. The lubricant can be fatty acid. The flame retardant can be antimony trioxide. The colorant can be antimony dioxide. The filler can be a calcium carbonate, a glass fiber, an asbestos fiber, a carbon fiber, a boron fiber, a copper disulfide, a glimmer, an asbestos, a silica, a silicate, a metal oxide, a carbon black, a glass bead or a wood flour. The antistatic agent can be stearylaminopropyldimethyl-β-hydroxyethylamine nitrate. The light stabilizer can be salicylate, benzophenone, benzotriazole, substituted acryl, substituted acrylonitrile, triazine or organic complex. The foaming agent can be azodicarbonamide. Therefore, the characteristic of the plastic, such as the impact resistance, the weather resistance, the heat resistance, the fluidity, the elasticity, the corrosion resistance, the deformation resistance, the light transmittance, the latent modification, the antistatic property, the photolysis resistance, the specific gravity or the toughness can be changed by modifying the plastic properties, which is not limited thereto.

Each technical feature in the abovementioned plasticizer and the plastic composition can be configured in combination to achieve the corresponding effect.

The present disclosure provides a plastic product, which includes the abovementioned plastic composition. Further, the plastic product of the present disclosure can be a packaging film, a composite paper, a disposable container or a storage utensil, a disposable tableware, a food packaging, a binding tape, a rope, a wrapping film, an agricultural seedling cup and a plate, a mushroom space bag, a fertilizer bag, an agricultural tape or a belt, an agricultural tiling or a covering material, an agricultural plug tray, a vegetable and a fruit bagging, an agricultural border covering membrane, a surgical needle, a surgical membrane, a bone plate, a drug carrier, an inorganic mixture, a dental filling material, a biological suture anchor, a biological tissue scaffold, a bone nail, a biological tissue filler, a biological suture, a biological experimental petri dish, a biological experimental container, a biological experimental micropipette, a biological experimental centrifuge tube, an experimental glove, etc.

According to the abovementioned embodiments, the specific comparative examples and examples are provided below and described in detail with the drawings.

<1st comparative example>

The plasticizer of the 1st comparative example is acetyltributylcitrate, and the details of the plasticizer of the 1st comparative example are shown in Table 2A.

| Table 2A | |
|---|---|
| Reactant A1 | - |
| Reactant A2 | - |
| Reactant B | - |
| Product | Acetyltributylcitrate |
| Structure | 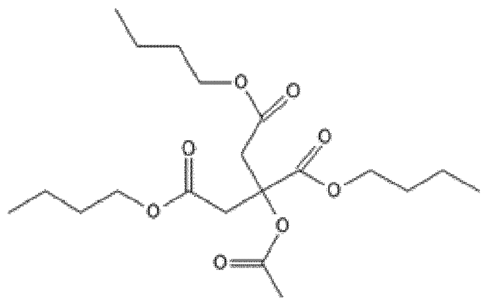 |
| Structure schematic | |
| SMILES Notation | CCCCOC(=O)CC(CC(=O)OCCCC)(C(=O)OCCCC)OC(=O)C |
| XC | - |
| YC | - |
| Mw (g/mole) | 402 |
| Pt (°C) | - |

Table 2B shows the value of the biodegradation rate of the plasticizer of the 1st comparative example at different days.

| Table 2B | |
|---|---|
| Day | Biodegradation rate (%) |
| 0 | 0.00 |
| 14 | 41.68 |
| 24 | 53.43 |
| 35 | 57.78 |
| 48 | 67.74 |
| 55 | 69.28 |
| 63 | 79.62 |
| 77 | 83.19 |
| 87 | 84.93 |
| 97 | 89.30 |
| 105 | 88.47 |
| 111 | 88.73 |
| 121 | 88.21 |
| 131 | 89.76 |
| 142 | 91.03 |
| 152 | 90.36 |
| 160 | 89.99 |
| 170 | 86.38 |
| 180 | 84.45 |

<1st example>

The plasticizer of the 1st example is 1,4-bis(2-ethylbutyl) 2-aminobutanedioate, and the details of the plasticizer of the 1st example are shown in Table 3A.

| Table 3A | |
|---|---|
| Reactant A1 | 2-Ethyl-1-butanol |
| Reactant A2 | - |
| Reactant B | Aspartic acid |
| Product | 1,4-bis(2-ethylbutyl) 2-aminobutanedioate |
| Structure | 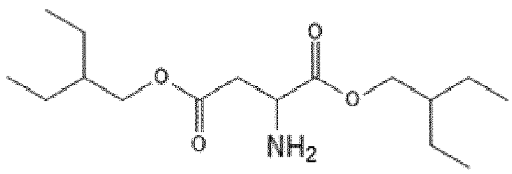 |

(continued)

| Table 3A | |
|---|---|
| Structure schematic | 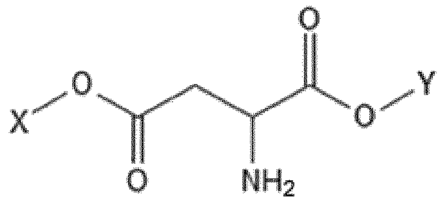 |
| SMILES Notation | CCC(CC)COC(=O)CC(N)C(=O)OCC(CC)CC |
| XC | 6 |
| YC | 6 |
| Mw (g/mole) | 301 |
| Pt (°C) | 200 |

Table 3B shows the value of the biodegradation rate of the plasticizer of the 1st example at different days.

| Table 3B | |
|---|---|
| Day | Biodegradation rate (%) |
| 0 | 0.00 |
| 14 | 12.06 |
| 24 | 32.26 |
| 35 | 43.38 |
| 48 | 61.70 |
| 55 | 67.41 |
| 63 | 70.17 |
| 77 | 77.40 |
| 87 | 77.85 |
| 97 | 78.48 |
| 105 | 78.48 |
| 111 | 78.48 |
| 121 | 79.86 |
| 131 | 81.38 |
| 142 | 84.03 |
| 152 | 84.78 |
| 160 | 86.07 |
| 170 | 89.10 |
| 180 | 93.16 |

<2nd example>

The plasticizer of the 2nd example is 1,4-dimethyl 2-aminobutanedioate, and the details of the plasticizer of the 2nd example are shown in Table 4.

| Table 4 | |
|---|---|
| Reactant A1 | Methanol |
| Reactant A2 | - |
| Reactant B | Aspartic acid |
| Product | 1,4-dimethyl 2-aminobutanedioate |
| Structure | 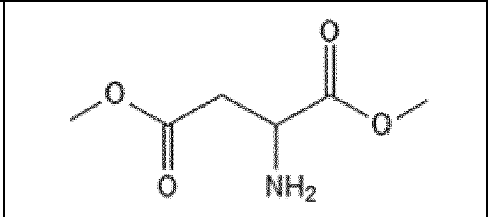 |
| Structure schematic | 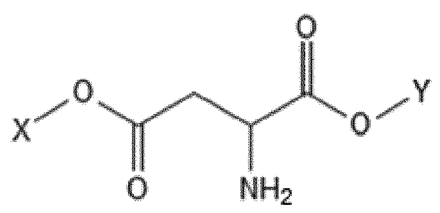 |
| SMILES Notation | COC(=O)CC(N)C(=O)OC |
| XC | 1 |
| YC | 1 |
| Mw (g/mole) | 162 |
| Pt (°C) | - |

<3rd example>

The plasticizer of the 3rd example is 1-methyl-4-octyl 2-aminobutanedioate, and the details of the plasticizer of the 3rd example are shown in Table 5.

| Table 5 | |
|---|---|
| Reactant A1 | 1-octanol |
| Reactant A2 | Methanol |
| Reactant B | Aspartic acid |
| Product | 1-methyl-4-octyl 2-aminobutanedioate |
| Structure | 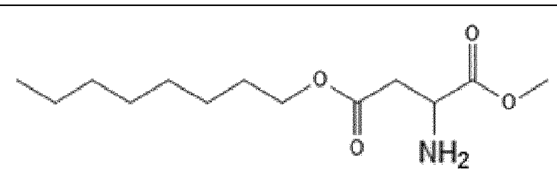 |
| Structure schematic | 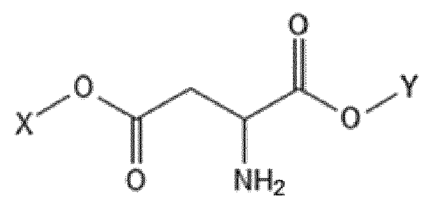 |
| SMILES Notation | CCCCCCCCOC(=O)CC(N)C(=O)OC |

(continued)

| Table 5 | |
|---|---|
| XC | 8 |
| YC | 1 |
| Mw (g/mole) | 259 |
| Pt (°C) | - |

<4th example>

The plasticizer of the 4th example is 1,4-bis(3-methylpentan-3-yl) 2-aminobutanedioate, and the details of the plasticizer of the 4th example are shown in Table 6.

| Table 6 | |
|---|---|
| Reactant A1 | 3-methyl-3-pentanol |
| Reactant A2 | - |
| Reactant B | Aspartic acid |
| Product | 1,4-bis(3-methylpentan-3-yl) 2-aminobutanedioate |
| Structure | 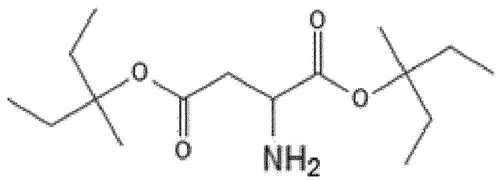 |
| Structure schematic | 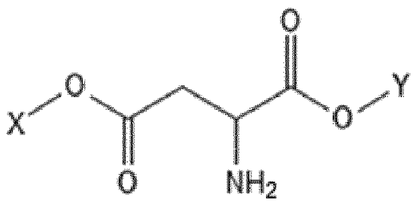 |
| SMILES Notation | CCC(C)(CC)OC(=O)CC(N)C(=O)OC(C)(CC)CC |
| XC | 6 |
| YC | 6 |
| Mw (g/mole) | 301 |
| Pt (°C) | - |

<5th Example>

The plasticizer of the 5th example is 1,4-bis(4-hydroxybutyl) 2-aminobutanedioate, and the details of the plasticizer of the 5th example are shown in Table 7.

| Table 7 | |
|---|---|
| Reactant A1 | 1,4-butanedio |
| Reactant A2 | - |
| Reactant B | Aspartic acid |
| Product | 1,4-bis(4-hydroxybutyl) 2-aminobutanedioate |

(continued)

| Table 7 | |
|---|---|
| Structure | 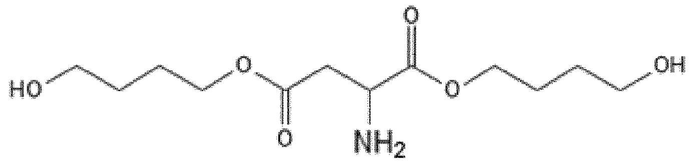 |
| Structure schematic | 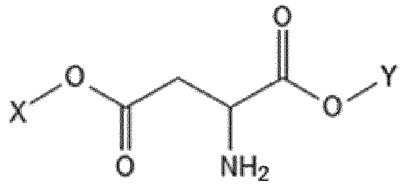 |
| SMILES Notation | NC(CC(=O)OCCCCO)C(=O)OCCCCO |
| XC | 4 |
| YC | 4 |
| Mw (g/mole) | 277 |
| Pt (°C) | - |

<6th example>

The plastic composition of the 6th example includes the plastic and the plasticizer, and the plastic contained in the plastic composition is polybutylene succinate, and the plasticizer contained in the plastic composition is 1,4-bis(2-ethylbutyl) 2-aminobutanedioate, wherein the details of 1,4-bis(2-ethylbutyl) 2-aminobutanedioate are shown in Table 3A, and will not be described herein. Table 8 shows the components and the contents of the plastic composition of the 6th example.

| Table 8 | | |
|---|---|---|
| Plastic | Component | Polybutylene succinate |
| | Weight percentage (%) | 98.40 |
| | | |
| Plasticizer | Component | 1,4-bis(2-ethylbutyl) 2-aminobutanedioate |
| | Weight percentage (%) | 1.60 |

<7th example>

The plastic composition of the 7th example includes the plastic and the plasticizer, and the plastic contained in the plastic composition is polybutylene succinate, and the plasticizer contained in the plastic composition is 1,4-bis(2-ethylbutyl) 2-aminobutanedioate, wherein the details of 1,4-bis(2-ethylbutyl) 2-aminobutanedioate are shown in Table 3A, and will not be described herein. Table 9 shows the components and the contents of the plastic composition of the 7th example.

| Table 9 | | |
|---|---|---|
| Plastic | Component | Polybutylene succinate |
| | Weight percentage (%) | 95.00 |
| | | |

(continued)

| Table 9 | | |
|---|---|---|
| Plasticizer | Component | 1,4-bis(2-ethylbutyl) 2-aminobutanedioate |
| | Weight percentage (%) | 5.00 |

<8th example>

The plastic composition of the 8th example includes the plastic and the plasticizer, and the plastic contained in the plastic composition is polybutylene succinate, and the plasticizer contained in the plastic composition is 1,4-bis(2-ethylbutyl) 2-aminobutanedioate, wherein the details of 1,4-bis(2-ethylbutyl) 2-aminobutanedioate are shown in Table 3A, and will not be described herein. Table 10 shows the components and the contents of the plastic composition of the 8th example.

| Table 10 | | |
|---|---|---|
| Plastic | Component | Polybutylene succinate |
| | Weight percentage (%) | 92.50 |
| | | |
| Plasticizer | Component | 1,4-bis(2-ethylbutyl) 2-aminobutanedioate |
| | Weight percentage (%) | 7.50 |

<9th example>

The plastic composition of the 9th example includes the plastic and the plasticizer, and the plastic contained in the plastic composition is polybutylene succinate, and the plasticizer contained in the plastic composition is 1,4-bis(2-ethylbutyl) 2-aminobutanedioate, wherein the details of 1,4-bis(2-ethylbutyl) 2-aminobutanedioate are shown in Table 3A, and will not be described herein. Table 11 shows the components and the contents of the plastic composition of the 9th example.

| Table 11 | | |
|---|---|---|
| Plastic | Component | Polybutylene succinate |
| | Weight percentage (%) | 90.00 |
| | | |
| Plasticizer | Component | 1,4-bis(2-ethylbutyl) 2-aminobutanedioate |
| | Weight percentage (%) | 10.00 |

<10th example>

The plastic composition of the 10th example includes the plastic and the plasticizer, and the plastic contained in the plastic composition is polybutylene succinate, and the plasticizer contained in the plastic composition is 1,4-bis(2-ethylbutyl) 2-aminobutanedioate, wherein the details of 1,4-bis(2-ethylbutyl) 2-aminobutanedioate are shown in Table 3A, and will not be described herein. Table 12 shows the components and the contents of the plastic composition of the 10th example.

| Table 12 | | |
|---|---|---|
| Plastic | Component | Polybutylene succinate |
| | Weight percentage (%) | 85.00 |
| | | |

(continued)

| Table 12 | | |
|---|---|---|
| Plasticizer | Component | 1,4-bis(2-ethylbutyl) 2-aminobutanedioate |
| | Weight percentage (%) | 15.00 |

<Cell Viability Test>

The 1st example is used as the experimental group to perform the cell viability test. Specifically, according to ISO10993-5 standard method, the mouse lung fibroblast (L929 cell line) is used as the test cell, and the composition of the test culture medium is 90% MEM culture medium, 10% fetal bovine serum (FBS) and additional 1% antibiotic (penicillin-streptomycin). Then, the ethanol with the final concentration of 0.1% is used as the solvent to dissolve the tested plasticizer in the test culture medium. The control group of the cell viability test is the test culture solution only added the ethanol with the final concentration of 0.1%. In the experimental group, the plasticizer of the 1st example with the concentration of 0.1 mM or 0.3 mM is added to the test culture solution, respectively, and the concentration of the ethanol in the test culture solution of the control group and the experimental group are the same, the test condition is the 37°C environment containing 5% carbon dioxide placed for 24 hours. After the test finished, each group is tested with MTT assay, the detection wavelength is the optical density at 570 nm ($OD_{570}$) and the optical density at 650 nm ($OD_{650}$), and the cell viability is calculated accordingly. The formula for calculating the cell viability is: (the average value of $OD_{570}$ and $OD_{650}$ in the experimental group)/(the average value of three repetitions of (the average value of $OD_{570}$ and $OD_{650}$ in the control group))×100%. The cell viability is used to determine whether the group has the cytotoxicity, and when the cell viability is more than 70%, it is considered that this group is non-cytotoxic.

Table 13 shows the cell viability of the results of the plasticizer of the 1st example in the concentration of 0.1 mM or 0.3 mM.

| Table 13 | | |
|---|---|---|
| | 0.1 mM | 0.3 mM |
| 1st example | 99.10% | 81.90% |

<Plasticizer Precipitation Test>

The test is to simulate the use of the plastic composition in the daily life to perform the plasticizer precipitation test and using the different solutions and temperatures for testing. The experimental steps as follows. (1) Preparing the thermostat controller and setting it at 60°C and 95°C. (2) Preparing the pure water, 4% acetic acid aqueous solution, 20% ethanol aqueous solution and n-hexane. (3) The plastic composition of the 6th example is soaked in the six different solutions and heated at the constant temperature to simulate various use environments of the plastic composition. The soaking solution and the temperature are 60°C of pure water for 30 minutes (experimental group A), 95°C of pure water for 30 minutes (experimental group B), 60°C of 4% acetic acid aqueous solution for 30 minutes (experimental group C), 95°C of 4% acetic acid aqueous solution for 30 minutes (experimental group D), 60°C of 20% ethanol aqueous solution for 30 minutes (experimental group E), 25°C of n-hexane for 60 minutes (experimental group F), respectively. (4) Measuring the concentration of the plasticizer of 1,4-bis(2-ethylbutyl) 2-aminobutanedioate in the abovementioned experiment group A to experiment group F, so as to determine whether 1,4-bis(2-ethylbutyl) 2-aminobutanedioate is precipitated due to the soaking treatment, and the precipitation refers to the release of the plasticizer in the plastic composition. Next, the soaked aqueous solutions of the plastic compositions in the abovementioned experiment group A to experiment group F are measured by LC-MS method, and 0.01% of 1,4-bis(2-ethylbutyl) 2-aminobutanedioate aqueous solution is used as the control group A to confirm whether the plastic composition will precipitate the plasticizer, wherein the threshold of the measurement is 100 ppm (about 0.33 mM).

Fig. 1 shows the results of LS-MS of the control group A. Fig. 2 shows the results of LS-MS of the experiment group E. Table 14 is the concentration of the precipitated plasticizer of the control group A and the experiment group A to the experiment group F.

| Table 14 | |
|---|---|
| | Plasticizer concentration (ppm) |
| Control group A | ≥ 100 |
| Experiment group A | < 100 |
| Experiment group B | < 100 |
| Experiment group C | < 100 |
| Experiment group D | < 100 |
| Experiment group E | < 100 |
| Experiment group F | < 100 |

The detection results of Fig. 1, Fig. 2 and Table 14 can be observed that the control group A has the peak value to produce, and the aqueous solution soaked with the plastic composition of the experiment group A to the experiment group F will not have the peak value to produce. It means that the plastic composition will not precipitate the plasticizer that greater than or equal to 100 ppm or will not precipitate plasticizer, so it has the better safety. Furthermore, due to the LS-MS results of the experiment group A to the experiment group F do not have the peak value, so that only the result of the experiment group E is shown.

Furthermore, it is known by the plasticizer precipitation test and the cell viability test of the present disclosure, the plasticizer of 1,4-bis(2-ethylbutyl) 2-aminobutanedioate in the 6th example has the precipitation amount in daily use is less than 100 ppm (about 0.33 mM), and 1,4-bis(2-ethylbutyl) 2-aminobutanedioate of 0.3 mM will not be toxic to the cells. It is presumed that the plasticizer of 1,4-bis(2-ethylbutyl) 2-aminobutanedioate is not easy to precipitate into the aqueous solution, and the plasticizer of 1,4-bis(2-ethylbutyl) 2-aminobutanedioate will not cause the obvious harm to the human body, so the plasticizer and the plastic composition of the present disclosure have the excellent safety.

## Claims

**1.** A plasticizer, which is an amino acid derivative and has a biodegradability, **characterized in** comprising:

a central structure and at least one side chain structure, wherein the central structure is an aspartic acid, the at least one side chain structure comprises a first side chain structure and a second side chain structure, and the plasticizer has a structure represented by Formula (I):

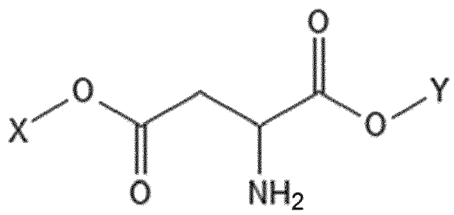

Formula (I),

wherein X is the first side chain structure, Y is the second side chain structure, a number of carbon atoms of the first side chain structure is XC, a number of carbon atoms of the second side chain structure is YC, and the following conditions are satisfied:

$$1 \leq XC \leq 7;$$

and

$$1 \leq YC \leq 7.$$

2. The plasticizer of claim 1, wherein at least one of X and Y is a saturated hydrocarbon chain.

3. The plasticizer of claims 1 or 2, wherein at least one of X and Y is a branched chain structure.

4. The plasticizer of any one of claims 1 to 3, wherein a number of carbon atoms of X is different from a number of carbon atoms of Y.

5. The plasticizer of any one of claims 1 to 4, wherein at least one of X and Y has a hydroxyl group.

6. The plasticizer of any one of claims 1 to 5, wherein a molecular weight of the plasticizer is Mw, and the following condition is satisfied:

$$300 \text{ g/mole} \leq Mw.$$

7. The plasticizer of any one of claims 1 to 6, wherein a pyrolysis temperature of the plasticizer is Pt, and the following condition is satisfied:

$$100^{\circ}C \leq Pt.$$

8. The plasticizer of any one of claims 1 to 7, wherein a biodegradation rate on a 14th day is Dr14, and the following condition of the biodegradation rate on the 14th day of the plasticizer is satisfied:

$$Dr14 \leq 15\%.$$

9. The plasticizer of any one of claims 1 to 8, wherein a biodegradation rate on a 35th day is Dr35, and the following condition of the biodegradation rate on the 35th day of the plasticizer is satisfied:

$$Dr35 \leq 45\%.$$

10. The plasticizer of any one of claims 1 to 9, wherein a biodegradation rate on a 180th day is Dr180, and the following condition of the biodegradation rate on the 180th day of the plasticizer is satisfied:

$$85\% \leq Dr180.$$

11. The plasticizer of any one of claims 1 to 10, wherein a cell viability is greater than or equal to 70% when a concentration of the plasticizer is 0.3 mM.

12. A plastic composition, **characterized in** comprising:

    at least one plastic; and
    the plasticizer of any one of claims 1 to 11.

13. The plastic composition of claim 12, wherein a weight percentage of the at least one plastic in the plastic composition is 85.00% to 99.99%, and a weight percentage of the plasticizer in the plastic composition is 0.01% to 15.00%.

14. The plastic composition of claims 12 or 13, wherein the at least one plastic comprises a biodegradable plastic.

15. The plastic composition of any one of claims 12 to 14, wherein the at least one plastic further comprises:
at least one non-biodegradable plastic, wherein a weight percentage of the at least one non-biodegradable plastic in the plastic composition is less than a weight percentage of the biodegradable plastic in the plastic composition.

**16.** A plastic product, **characterized in** comprising: the plastic composition of any one of claims 12 to 15.

**17.** A plasticizer, which is an amino acid derivative and has a biodegradability, **characterized in** comprising:

a central structure and at least one side chain structure, wherein the central structure is an aspartic acid, the at least one side chain structure comprises a first side chain structure and a second side chain structure, and the plasticizer has a structure represented by Formula (I):

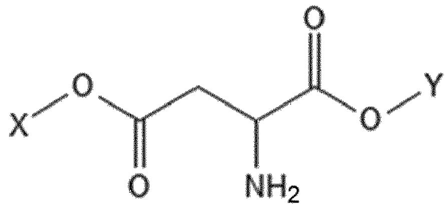

Formula (I),

wherein X is the first side chain structure, Y is the second side chain structure, and at least one of X and Y is a branched chain structure.

**18.** The plasticizer of claim 17, wherein a number of carbon atoms of the first side chain structure is XC, a number of carbon atoms of the second side chain structure is YC, and the following conditions are satisfied:

$$1 \leq XC \leq 10;$$

and

$$1 \leq YC \leq 10.$$

**19.** The plasticizer of claims 17 or 18, wherein at least one of X and Y is a saturated hydrocarbon chain.

**20.** The plasticizer of any one of claims 17 to 19, wherein a biodegradation rate on a 14th day is Dr14, and the following condition of the biodegradation rate on the 14th day of the plasticizer is satisfied:

$$Dr14 \leq 15\%.$$

**21.** The plasticizer of any one of claims 17 to 20, wherein a biodegradation rate on a 35th day is Dr35, and the following condition of the biodegradation rate on the 35th day of the plasticizer is satisfied:

$$Dr35 \leq 45\%.$$

**22.** The plasticizer of any one of claims 17 to 21, wherein a biodegradation rate on a 180th day is Dr180, and the following condition of the biodegradation rate on the 180th day of the plasticizer is satisfied:

$$85\% \leq Dr180.$$

**23.** A plastic composition, **characterized in** comprising:

at least one plastic; and
at least one plasticizer, wherein the plasticizer is an amino acid derivative and has a biodegradability, and the

plasticizer comprises a central structure and at least one side chain structure;
wherein a weight percentage of the at least one plastic in the plastic composition is 85.00% to 99.99%, and a weight percentage of the plasticizer in the plastic composition is 0.01% to 15.00%.

**24.** The plastic composition of claim 23, wherein at least one of the at least one side chain structure is a long chain structure with a number of carbon atoms ranging from 1 to 10.

**25.** The plastic composition of claims 23 or 24, wherein at least one of the at least one side chain structure is a saturated hydrocarbon chain.

**26.** The plastic composition of any one of claims 23 to 25, wherein at least one of the at least one side chain structure is a branched chain structure.

**27.** The plastic composition of any one of claims 23 to 26, wherein a biodegradation rate on a 14th day is Dr14, and the following condition of the biodegradation rate on the 14th day of the plasticizer is satisfied:

$$Dr14 \leq 15\%.$$

**28.** The plastic composition of any one of claims 23 to 27, wherein a biodegradation rate on a 35th day is Dr35, and the following condition of the biodegradation rate on the 35th day of the plasticizer is satisfied:

$$Dr35 \leq 45\%.$$

**29.** The plastic composition of any one of claims 23 to 28, wherein a biodegradation rate on a 180th day is Dr180, and the following condition of the biodegradation rate on the 180th day of the plasticizer is satisfied:

$$85\% \leq Dr180.$$

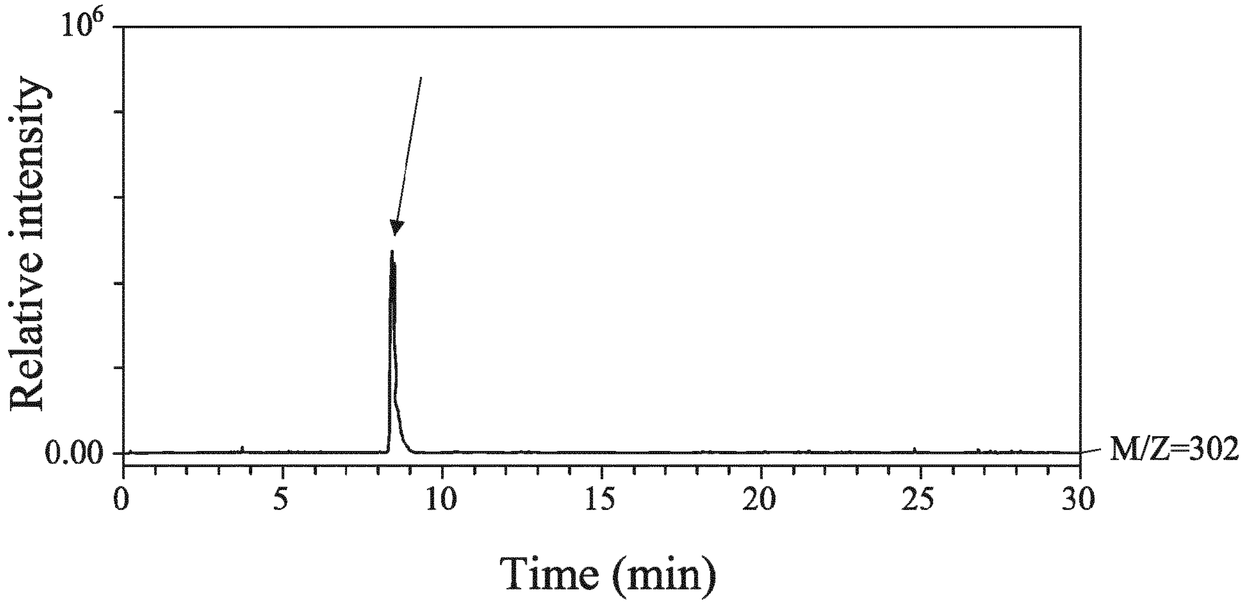
$10^6$
Relative intensity
0.00
M/Z=302
0
5
10
15
20
25
30
Time (min)

Fig. 1

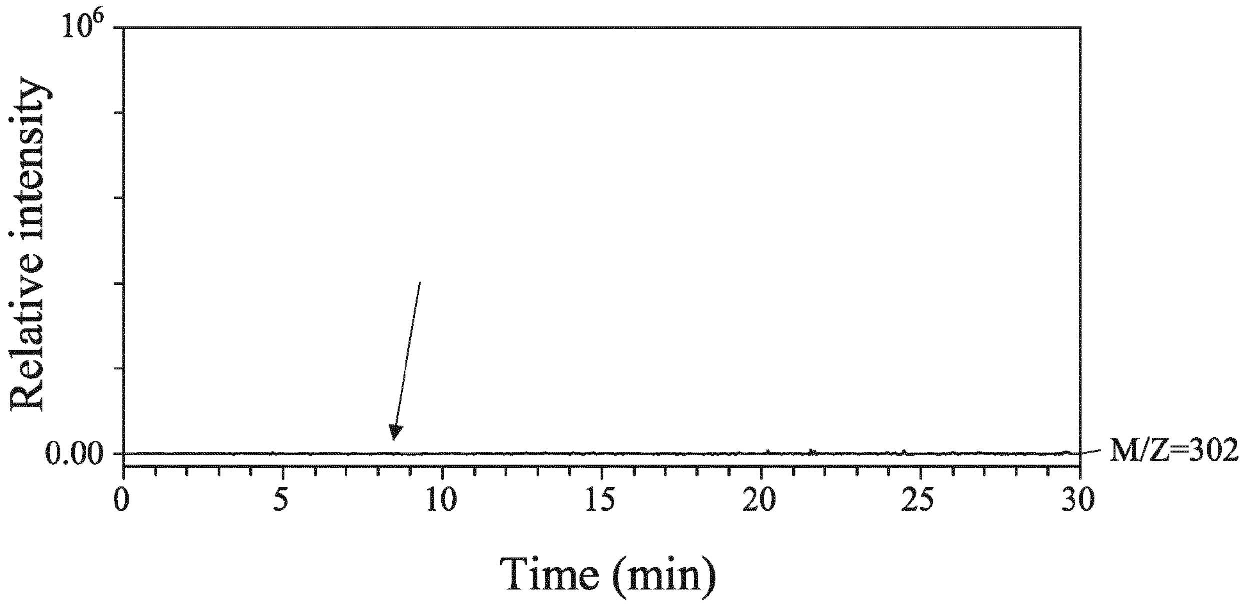
$10^6$
Relative intensity
0.00
M/Z=302
0
5
10
15
20
25
30
Time (min)

Fig. 2

Europäisches Patentamt
European Patent Office
Office européen des brevets

# EUROPEAN SEARCH REPORT

Application Number
EP 23 22 0171

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | STEIN T M ET AL: "Amino acids as plasticizers - II. Use of quantitative structure-property relationships to predict the behavior of monoammoniummonocarboxylate plasticizers in starch-glycerol blends", CARBOHYDRATE POLYMERS, APPLIED SCIENCE PUBLISHERS , LTD BARKING, GB, vol. 39, no. 1, 1 May 1999 (1999-05-01), pages 7-16, XP004160581, ISSN: 0144-8617, DOI: 10.1016/S0144-8617(98)00136-2<br>* abstract; figure 1; compounds 7,13-15,17-20 *<br>* chapter 1 *<br>----- | 23-26 | INV.<br>C07C69/00<br>C07C211/01<br>C08K5/00<br>C08K5/17 |
| X | WO 2019/172835 A1 (MEDIVIR AB [SE]) 12 September 2019 (2019-09-12)<br>* compounds I-34a *<br>----- | 1-3,6,7, 17-19 | |
| X | DATABASE REGISTRY [Online] Chemical Abstract Service, Columbus, Ohio, US; 1 October 2019 (2019-10-01), Anonymous: XP093150680, retrieved from STN Database accession no. 2376206-03-8<br>* abstract *<br>----- | 1-3,6,7, 17-19 | TECHNICAL FIELDS SEARCHED (IPC)<br>C09J<br>C07C<br>C08K<br>C08L |

-/--

The present search report has been drawn up for all claims

| Place of search | Date of completion of the search | Examiner |
|---|---|---|
| Munich | 12 April 2024 | Schütte, Maya |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 22 0171

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCHÖLLKOPF ULRICH ET AL: "Asymmetric Syntheses via Heterocyclic Intermediates; XII. Enantioselective Syntesis of (R)-[alpha] Amino Acids using tert-Leucine as Chiral Auxiliary Reagent", SYNTHESIS, no. 10, 1 October 1982 (1982-10-01), pages 861-864, XP093151107, Stuttgart DOI: 10.1055/s-1982-29977 * abstract; example 10e; table 2 * ----- | 1-4,6,7, 17-19 | |
| X | EP 3 825 354 A1 (LARGAN MEDICAL CO LTD [TW]) 26 May 2021 (2021-05-26) * abstract; claims 1,6; examples 17,29 * ----- | 5,12-16, 23-26 | |
| | | | TECHNICAL FIELDS SEARCHED (IPC) |

The present search report has been drawn up for all claims

2

| Place of search | Date of completion of the search | Examiner |
|---|---|---|
| Munich | 12 April 2024 | Schütte, Maya |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 22 0171

This annex lists the patent family members relating to the patent documents cited in the above-mentioned European search report. The members are as contained in the European Patent Office EDP file on
The European Patent Office is in no way liable for these particulars which are merely given for the purpose of information.

12-04-2024

| Patent document cited in search report | Publication date | Patent family member(s) | Publication date |
|---|---|---|---|
| WO 2019172835 A1 | 12-09-2019 | AU 2019232478 A1 | 15-10-2020 |
| | | CA 3094958 A1 | 12-09-2019 |
| | | CN 112105624 A | 18-12-2020 |
| | | EP 3762394 A1 | 13-01-2021 |
| | | JP 7337855 B2 | 04-09-2023 |
| | | JP 2021516700 A | 08-07-2021 |
| | | KR 20200141449 A | 18-12-2020 |
| | | US 2020399295 A1 | 24-12-2020 |
| | | WO 2019172835 A1 | 12-09-2019 |
| EP 3825354 A1 | 26-05-2021 | CN 112831091 A | 25-05-2021 |
| | | CN 116003877 A | 25-04-2023 |
| | | CN 116082703 A | 09-05-2023 |
| | | EP 3825354 A1 | 26-05-2021 |
| | | JP 2021085036 A | 03-06-2021 |
| | | TW 202120611 A | 01-06-2021 |
| | | TW 202212450 A | 01-04-2022 |
| | | TW 202334301 A | 01-09-2023 |
| | | US 2021155771 A1 | 27-05-2021 |

EPO FORM P0459

For more details about this annex : see Official Journal of the European Patent Office, No. 12/82